# EUROPÄISCHE PATENTANMELDUNG

(11) **EP 0 699 650 A1**
(43) Veröffentlichungstag der Anmeldung: **06.03.1996**
(21) Anmeldenummer: 95113005.3
(22) Anmeldetag: 18.08.1995
(51) Int. Cl.: C07C 17/358, C07C 25/08, C07C 25/10

(54) **Verbessertes Verfahren zur Isomerisierung von chlorierten Aromaten**

(30) Priorität: 31.08.1994 DE 4430950
(71) Anmelder: BAYER AG, D-51368 Leverkusen (DE)
(72) Erfinder: Rasp, Christian, Dr., D-51467 Bergisch Gladbach (DE); Waldmann, Helmut, Dr., D-51373 Leverkusen (DE)

(57) **Zusammenfassung**

Bei einem verbesserten Verfahren zur Isomerisierung von Mono-, Di- und/oder Polychloraromaten, bei dem die Aktivität und Lebensdauer der verwendeten Hilfsmittel hoch sind und das deshalb auch kontinuierlich durchgeführt werden kann, werden Mono-, Di- und/oder Polychloraromaten bei erhöhter Temperatur mit Aluminiumtrichlorid, einem Wasserspender und Chlorwasserstoff behandelt.

## Beschreibung

Die vorliegende Erfindung betrifft ein verbessertes Verfahren zur Isomerisierung von chlorierten Aromaten durch Behandlung mit Aluminiumchlorid und weiteren Hilfsmitteln.

Bei der Chlorierung von Aromaten erhält man bei der Anlagerung von 2 oder mehr Chloratomen nur bestimmte Isomere in bestimmten Verhältnissen zueinander. Durch Isomerisierung sind andere Isomere zugänglich oder kann höherer Bedarf an bestimmten Isomeren befriedigt werden.

Es sind eine Reihe solcher Isomerisierungsverfahren bekannt, bei denen beispielsweise Zeolithe, Alkaliamide und tert. Amine, Aluminiumchlorid/Natriumchlorid-Schmelzen oder diverse Salze als Hilfsmittel eingesetzt werden. Gemäß der DE-PS 947 304 und J.Org. Chem. 27 3449 (1962) wird basisches Aluminiumchlorid, d.h. Aluminiumchlorid und wenig Wasser, als Hilfsmittel für derartige Isomerisierungen vorgeschlagen. Die DE-AS 10 20 323 und die US-PS 2 920 109 nennen für den gleichen Zweck Aluminiumchlorid, gegebenenfalls mit Chlorwasserstoffzusatz. In Zhur. Obshchei Khim. 28 1637 (1958) und 31 292 (1960) wird ebenfalls über die Isomerisierung mit Aluminiumtrichlorid, gegebenenfalls mit Zusätzen von Metalloxiden und -salzen berichtet. Eine Arbeitsweise mit Aluminiumtrichlorid und Ameisensäure wird in der DE-OS 31 34 726 beschrieben.

Nachteilig bei allen diesen Verfahren ist, daß die Aktivität und Lebensdauer der verwendeten Hilfsmittel nicht optimal sind und deshalb eine kontinuierliche Arbeitsweise erschwert ist.

Es wurde nun ein Verfahren zur Isomerisierung von Mono-, Di- und/oder Polychloraromaten gefunden, das dadurch gekennzeichnet ist, daß man Mono-, Di- und/oder Polychloraromaten bei erhöhter Temperatur mit Aluminiumtrichlorid, einem Wasserspender und Chlorwasserstoff behandelt.

In das erfindungsgemäße Verfahren kann man z.B. Mono-, Di- und/oder Polychloraromaten der Formel (I) einsetzen 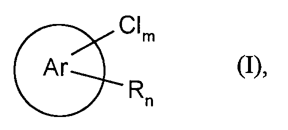
in der
- Ar: für einen Benzol-, Naphthalin- oder Diphenylether-Rest,
- R: für C₁-C₄-Alkyl, Mono- bis Trichlor-C₁-C₄-Alkyl, C₆-C₁₀-Aryl, Mono- bis Pentachlor-C₆-C₁₀-Aryl, C₇-C₁₄-Alkaryl oder Mono- bis Pentachlor-C₇-C₁₄-Alkaryl,
- m: für eine ganze Zahl von 1 bis 5 und
- n: für null oder eine ganze Zahl von 1 bis 4 steht,
wobei m nicht für 1 steht, wenn n für null steht und
wobei die Summe von n + m bei Ar = Benzolrest 6 und bei Ar = Naphthalinrest 8 nicht übersteigt.

Vorzugsweise steht in Formel (I)
- Ar: für einen Benzol- oder Naphthalinrest,
- R: C₁-C₄-Alkyl,
- m: für eine ganze Zahl von 1 bis 4 und
- n: für null, 1 oder 2,
wobei m nicht für 1 steht, wenn n für null steht.

Besonders bevorzugt setzt man in die erfindungsgemäße Isomerisierung 1,2-Dichlorbenzol, 1,4-Dichlorbenzol, 1,2-/1,4-Dichlorbenzolgemische, 1,2-/1,3-/1,4-Dichlorbenzolgemische außerhalb des Isomerisierungsgleichgewichts, 1,2,4-Trichlorbenzol und 1,2,4-Trichlorbenzol-enthaltende Trichlorbenzolgemische außerhalb des Isomerisierungsgleichgewichts ein.

Geeignete Temperaturen für das erfindungsgemäße Verfahren sind z.B. solche im Bereich von 130°C bis zum Siedepunkt des Reaktionsgemisches. Bevorzugt arbeitet man beim Einsatz von Kristallwasser enthaltenden Salzen bei 135 bis 165°C und beim Einsatz von organischen Wasserspendern bei 165 bis 195°C.

Bezogen auf eingesetzte Mono-, Di- und/oder Polychloraromaten kann man beispielsweise 0,1 bis 30 Mol-% Aluminiumtrichlorid einsetzen. Vorzugsweise liegt diese Menge bei 0,5 bis 20 Mol-%.

Bei dem Wasserspender kann es sich z.B. um ein Kristallwasser enthaltendes Salz handeln, das bei den angewendeten Temperaturen Wasser abgibt, beispielsweise um entsprechende Alkali- oder Erdalkalisulfate sowie Aluminiumchloride. Vorzugsweise wird wasserhaltiges Natrium-, Magnesium- oder Kalziumsulfat (Gips) eingesetzt, besonders bevorzugt Glaubersalz (Na₂SO₄ x 10 H₂O), Bittersalz (MgSO₄ x 7 H₂O) und Aluminiumtrichlorid-Hexahydrat.

Bei dem Wasserspender kann es sich auch um eine organische Verbindung handeln, die Kristallwasser enthält, das bei den angewendeten Temperaturen abgegeben wird und/oder um eine organische Verbindung, die sich bei den angewendeten Temperaturen unter Wasserabspaltung zersetzt. Beispiele sind Ameisensäure, Oxalsäure und Oxalsäuredihydrat. Bevorzugt ist hier Ameisensaure.

Der Wasserspender kann beispielsweise in solchen Mengen eingesetzt werden, daß während der Isomerisierung daraus 0,1 bis 30 Mol-%, vorzugsweise 0,5 bis 20 Mol-%, Wasser (bezogen auf Mono-, Di- und/oder Polychloraromaten) freigesetzt werden. Besonders bevorzugt werden Wasserspender in solchen Mengen eingesetzt, daß während der Isomerisierung daraus 0,8 bis 1,2 Mol Wasser, bezogen auf 1 Mol eingesetztes Aluminiumtrichlorid freigesetzt werden.

Den Chlorwasserstoff kann man beispielsweise mit einem Partialdruck von 0,01 bis 5 bar einsetzen. Bevorzugt sind Chlorwasserstoff-Partialdrucke von 0,05 bis 2 bar, insbesondere 0,1 bis 1 bar.

Die Isomerisierungreaktion ist dann beendet, wenn sich im Isomerisierungsgemisch die isomere Verteilung nicht mehr ändert. Das ist im allgemeinen nach 5 bis 30 Stunden der Fall. Aus ökonomischen Gründen kann es in manchen Fällen angezeigt sein, die erfindungsgemäße Isomerisierung nicht bis zur vollständigen Einstellung des Isomerisierungsgleichgewichts durchzuführen.

Die nach der Isomerisierungsreaktion vorliegenden Reaktionsgemische kann man beispielsweise aufarbeiten, indem man sie zunächst filtriert oder dekantiert und dann aus der abgetrennten flüssigen Phase durch Destillation die einzelnen isomeren chlorierten Aromaten gewinnt. Gegebenenfalls kann man den Filterkuchen mit Wasser versetzen und restliche isomer chlorierte Aromaten durch Extraktion, z.B. mit Chloroform, abtrennen.

Die erfindungsgemäße Isomerisierung kann man diskontinuierlich, teilkontinuierlich und kontinuierlich durchführen. Bei diskontinuierlicher Arbeitsweise kann man z.B. in einem druckfesten Rührgefäß Mono-, Di- und/oder Polychloraromaten, Aluminiumtrichlorid und Wasserspender vorlegen, dann erwärmen und beim gewünschten Druck unter Rühren Chlorwasserstoffgas einleiten.

Bei teilkontinuierlicher Arbeitsweise kann man z.B. in einem druckfesten Rührgefäß Mono-, Di- und/oder Polychloraromaten, Aluminiumtrichlorid und Wasserspender vorlegen, dann erwärmen und beim gewünschten Druck unter Rühren Chlorwasserstoffgas einleiten. Nach einiger Zeit, vorzugsweise wenn die Isomerisierung weitgehend beendet ist, kann man dann abkühlen, die Chlorwasserstoffzufuhr unterbrechen, den Rührer abstellen und die flüssige Phase, z.B. durch Dekantieren, weitgehend entfernen und durch Destillation aufarbeiten. Den im Reaktor verbliebenen Rückstand kann man dann wieder mit Mono-, Di- und/oder Polychloraromaten, Aluminiumtrichlorid und Wasserspender versetzen und einen neuen Reaktionszyklus beginnen. Bei derartigen neuen Reaktionszyklen, auch bei mehrfacher Wiederholung, ist erneutes Einleiten von Chlorwasserstoffgas nicht mehr erforderlich.

Bei kontinuierlicher Arbeitsweise kann man z.B. in einem Reaktor Mono-, Di- und/oder Polychloraromaten vorlegen, erwärmen, unter Rühren beim gewünschten Partialdruck Chlorwasserstoffgas einleiten und Aluminiumtrichlorid und Wasserspender, suspendiert in Mono-, Di- und/oder Polychloraromaten, zudosieren. Nach einer Anfahrtzeit kann man dann Isomerisierungsgemisch kontinuierlich entnehmen und Mono-, Di- und/oder Polychloraromaten zusammen mit Aluminiumtrichlorid und Wasserspender kontinuierlich zuführen. Chlorwasserstoffgas kann man im Kreis führen.

Für die diskontinuierliche, teilkontinuierliche und kontinuierliche Arbeitsweise sind natürlich auch andere Ausführungsformen möglich.

Das erfindungsgemäße Isomerisierungsverfahren hat den Vorteil, daß die angewendete Hilfsmittelkombination eine hohe Aktivität und lange Lebensdauer aufweist, was sie für kontinuierliche Verfahren besonders geeignet macht.

Bei den nachfolgenden Beispielen wurden die Reaktionsgemische analysiert, indem entnommene Proben zunächst mit wäßriger Salzsäure gewaschen und dann mit Methylenchlorid extrahiert wurden. Die Methylenchlorid-Phasen wurden gaschromatografisch untersucht.

### Beispiele

### Beispiel 1

In einem 1 l-Glas-Reaktor wurden 1 Mol 1,4-Dichlorbenzol, 0,01 Mol Aluminiumtrichlorid und 0,014 Mol Bittersalz vorgelegt und bei Normaldruck unter starkem Rühren 2 l/Stunde Chlorwasserstoffgas eingeleitet. Innerhalb einer halben Stunde wurde auf 150°C aufgeheizt und in Abständen Proben aus dem Reaktionsgemisch entnommen und analysiert. Die Einzelheiten sind aus Tabelle 1 ersichtlich.

**Tabelle 1**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 100 | 0 |
| 1 | 0,8 | 99,2 | 0 |
| 3 | 5,5 | 94,4 | 0,10 |
| 5 | 10,9 | 89,0 | 0,17 |
| 7 | 15,5 | 84,3 | 0,22 |
| 9 | 19,7 | 80,0 | 0,28 |
| 11 | 22,0 | 77,5 | 0,43 |
| 13 | 24,6 | 74,9 | 0,46 |
| 25 | 31,5 | 67,7 | 0,72 |
| DCB = Dichlorbenzol | | | |

### Beispiel 2 (zum Vergleich)

Es wurde verfahren wie in Beispiel 1, jedoch wurde kein Chlorwasserstoff eingeleitet. Einzelheiten sind aus Tabelle 2 ersichtlich.

**Tabelle 2**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 100 | 0 |
| 1 | 1,5 | 98,3 | 0,17 |
| 3 | 7,9 | 91,9 | 0,20 |
| 5 | 12,7 | 87,1 | 0,23 |
| 7 | 15,5 | 84,2 | 0,27 |
| 9 | 16,7 | 83,0 | 0,30 |
| 11 | 16,8 | 82,8 | 0,33 |
| 13 | 17,0 | 82,6 | 0,35 |
| 25 | 18,9 | 80,6 | 0,41 |
| DCB = Dichlorbenzol | | | |

### Beispiel 3

Es wurde verfahren wie in Beispiel 1, jedoch wurde statt 1,4-Dichlorbenzol 1 Mol 1,2-Dichlorbenzol eingesetzt. Einzelheiten sind aus Tabelle 3 ersichtlich.

**Tabelle 3**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 0 | 100 |
| 1 | 3,4 | 0,7 | 95,9 |
| 3 | 8,7 | 1,2 | 90,1 |
| 5 | 10,8 | 1,5 | 87,7 |
| 7 | 11,5 | 1,5 | 87,0 |
| 21 | 14,1 | 2,1 | 83,4 |
| DCB = Dichlorbenzol | | | |

### Beispiel 4 (zum Vergleich)

Es wurde verfahren wie in Beispiel 3, jedoch wurde kein Chlorwasserstoffgas eingeleitet. Einzelheiten sind aus Tabelle 4 ersichtlich.

**Tabelle 4**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 0 | 100 |
| 1 | 2,0 | 0,8 | 97,2 |
| 3 | 2,1 | 0,9 | 97,0 |
| 5 | 2,1 | 1,0 | 96,9 |
| 7 | 2,2 | 1,0 | 96,8 |
| 21 | 2,4 | 1,3 | 96,3 |
| DCB = Dichlorbenzol | | | |

### Beispiel 5

Es wurde verfahren wie in Beispiel 3, jedoch wurden 0,1 Mol Aluminiumtrichlorid und anstelle von Bittersalz 0,1 Mol Ameisensäure eingesetzt und auf 180°C aufgeheizt. Einzelheiten sind aus der Tabelle 5 ersichtlich.

**Tabelle 5**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 0 | 100 |
| 1 | 15,3 | 2,1 | 82,6 |
| 3 | 39,5 | 14,5 | 46,0 |
| 5 | 45,0 | 18,9 | 36,0 |
| 7 | 46,5 | 19,7 | 33,6 |
| 21 | 59,5 | 30,0 | 8,5 |
| DCB = Dichlorbenzol | | | |

### Beispiel 6 (zum Vergleich)

Es wurde verfahren wie in Beispiel 5, jedoch wurde kein Chlorwasserstoffgas eingeleitet. Einzelheiten sind aus der Tabelle 6 ersichtlich.

**Tabelle 6**

| Reaktionszeit (Stunden) | 1,3-DCB (Gew.-%) | 1,4-DCB (Gew.-%) | 1,2-DCB (Gew.-%) |
|---|---|---|---|
| 0 | 0 | 0 | 100 |
| 1 | 7,1 | 0,5 | 92,4 |
| 3 | 14,5 | 2,1 | 83,4 |
| 5 | 17,0 | 3,5 | 79,4 |
| 7 | 19,5 | 4,9 | 75,5 |
| 21 | 35,0 | 11,6 | 52,4 |
| DCB = Dichlorbenzol | | | |

### Beispiel 7

In einem 1-l-Glas-Reaktor wurden 1 Mol 1,2,4-Trichlorbenzol, 0,1 Mol Aluminiumtrichlorid und 0,1 Mol Ameisensäure vorgelegt und bei Normaldruck unter starkem Rühren 2 l/h Chlorwasserstoff eingeleitet. Innerhalb einer halben Stunde wurde auf 185°C aufgeheizt. Einzelheiten sind aus Tabelle 7 ersichtlich.

**Tabelle 7**

| Reaktionszeit (Stunden) | Isomerisierung zu 1,2,3-TCB (Gew.-%) | 1,3,5-TCB (Gew.-%) |
|---|---|---|
| 0 | 0 | 0 |
| 1 | 5,2 | 1,1 |
| 2 | 6,9 | 3,0 |
| 3 | 7,4 | 4,4 |
| 4 | 7,6 | 5,3 |
| 5 | 7,7 | 6,1 |
| 6 | 7,8 | 6,8 |
| 7 | 7,9 | 7,4 |
| TCB = Trichlorbenzol | | |

### Beispiel 8 (zum Vergleich)

Es wurde gearbeitet wie in Beispiel 7, jedoch wurde kein Chlorwasserstoff eingeleitet. Einzelheiten sind aus der Tabelle 8 ersichtlich.

**Tabelle 8**

| Reaktionszeit (Stunden) | Isomerisierung zu 1,2,3-TCB (Gew.-%) | 1,3,5-TCB (Gew.-%) |
|---|---|---|
| 0 | 0 | 0 |
| 1 | 2,3 | 1,9 |
| 2 | 4,3 | 3,6 |
| 3 | 5,6 | 4,5 |
| 4 | 6,6 | 5,3 |
| 5 | 6,9 | 5,9 |
| 6 | 7,1 | 6,3 |
| 7 | 7,2 | 6,7 |
| TCB = Trichlorbenzol | | |

### Beispiel 9

1 Mol 1,4-Dichlorbenzol, 0,1 Mol Aluminiumtrichlorid und 0,1 Mol Ameisensäure wurden bei 170°C 2 Stunden miteinander verrührt. Dann wurde bei 150°C für 5 Stunden unter starkem Rühren 2 l/h Chlorwasserstoff eingeleitet, anschließend auf 50°C abgekühlt und die Hauptmenge der flüssigen Phase durch Dekantieren abgetrennt. Danach wurden dem Reaktor wieder 1 Mol 1,4-Dichlorbenzol und 0,02 Mol Aluminiumtrichlorid zugeführt und unter starkem Rühren bei 150°C 2 l/h Chlorwasserstoff für 2 Stunden eingeleitet. Danach wurden die Schritte Abkühlen, Dekantieren und erneutes Zuführen von 1,4-Dichlorbenzol, Aluminiumtrichlorid und Chlorwasserstoff mehrmals wiederholt. Ameisensäure mußte nicht nachdosiert werden, weil infolge des Hantierens an der Luft Wasser in Form von Luftfeuchtigkeit in ausreichender Menge ergänzt wurde. Einzelheiten sind aus Tabelle 9 ersichtlich.

**Tabelle 9**

| Reaktionszyklus | Reaktionszeit im jeweiligen Zyklus[h] | 1,4-DCB (Gew.-%) | 1,3-DCB (Gew.-%) | 1,2-DCB Gew.-%) |
|---|---|---|---|---|
| Stammansatz | 0 | 100 | 0 | 0 |
| | 5 | 42,7 | 53,2 | 4,1 |
| 1. Wiederholung | 0 | 89,7 | 9,6 | 0,7 |
| | 2 | 43,9 | 52,3 | 3,8 |
| 2. Wiederholung | 0 | 92,4 | 7,1 | 0,5 |
| | 2 | 44,4 | 52,0 | 3,6 |
| 3. Wiederholung | 0 | 92,5 | 7,1 | 0,4 |
| | 2 | 51,0 | 46,4 | 2,6 |
| 4. Wiederholung | 0 | 91,1 | 8,4 | 0,5 |
| | 2 | 61,7 | 36,7 | 1,6 |
| 5. Wiederholung | 0 | 90,5 | 9,1 | 0,4 |
| | 2 | 69,8 | 20,1 | 1,1 |
| DCB = Dichlorbenzol | | | | |

### Beispiel 10

1 Mol 1,4-Dichlorbenzol, 0,083 Mol Aluminiumtrichlorid und 0,017 Mol Aluminiumtrichlorid-Hexahydrat wurden vermischt und 2 Stunden bei 150°C zur Reaktion gebracht. Anschließend wurde auf 50°C abgekühlt, die Hauptmenge der flüssigen Phase durch Dekantieren abgetrennt. Danach wurden dem Reaktor wieder 1 Mol 1,4-Dichlorbenzol, 0,0175 Mol Aluminiumtrichlorid und 0,0025 Mol Aluminiumtrichlorid-Hexahydrat zugeführt und erneut für 2 Stunden bei 150°C zur Reaktion gebracht. Danach wurden die Schritte Abkühlen, Dekantieren und Zuführen von 1,4-Dichlorbenzol, Aluminiumtrichlorid und Aluminiumtrichlorid-Hexahydrat wiederholt. Chlorwasserstoff wurde nicht zugegeben, weil infolge des Hantierens an der Luft Wasser in Form von Luftfeuchtigkeit hinzutrat und aus AlCl₃ ausreichend HCl freisetzte.

Einzelheitenn sind aus Tabelle 10 ersichtlich.

**Tabelle 10**

| Reaktionszyklus | Reaktionszeit im jeweiligen Zyklus [h] | 1,4-DCB (Gew.-%) | 1,3-DCB (Gew.-%) | 1,2-DCB Gew.-%) |
|---|---|---|---|---|
| Stammansatz | 0 | 100 | 0 | 0 |
| | 2 | 43,8 | 53,7 | 2,5 |
| 1. Wiederholung | 0 | 88,4 | 11,1 | 0,5 |
| | 2 | 43,1 | 53,8 | 3,1 |
| 2. Wiederholung | 0 | 89,7 | 10,0 | 0,3 |
| | 2 | 42,9 | 54,1 | 3,0 |
| 3. Wiederholung | 0 | 87,2 | 12,1 | 0,7 |
| | 2 | 47,3 | 50,2 | 2,5 |
| 4. Wiederholung | 0 | 88,1 | 11,3 | 0,6 |
| | 2 | 47,9 | 49,8 | 2,3 |
| 5. Wiederholung | 0 | 83,7 | 15,4 | 0,9 |
| | 2 | 43,1 | 53,8 | 3,1 |

## Patentansprüche

1. Verfahren zur Isomerisierung von Mono-, Di- und/oder Polychloraromaten, dadurch gekennzeichnet, daß man Mono-, Di- und/oder Polychloraromaten bei erhöhter Temperatur mit Aluminiumtrichlorid, einem Wasserspender und Chlorwasserstoff behandelt.

2. Verfahren nach Anspruch 1, dadurch gekennzeichnet, daß man Di- und/oder Polychloraromaten der Formel (I) einsetzt 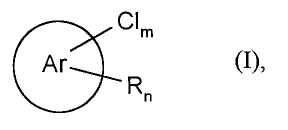 in der
Ar für einen Benzol-, Naphthalin- oder Diphenylether-Rest,
R für C₁-C₄-Alkyl, Mono- bis Trichlor-C₁-C₄-Alkyl, C₆-C₁₀-Aryl, Mono- bis Pentachlor-C₆-C₁₀-Aryl, C₇-C₁₄-Alkaryl oder Mono- bis Pentachlor-C₇-C₁₄-Alkaryl,
m für eine ganze Zahl von 1 bis 5 und
n für null oder eine ganze Zahl von 1 bis 4 steht,
wobei m nicht für 1 steht, wenn n für null steht und wobei die Summe von n + m bei Ar = Benzolrest 6 und bei Ar = Naphthalinrest 8 nicht übersteigt.

3. Verfahren nach Ansprüchen 1 bis 2, dadurch gekennzeichnet, daß man es bei Temperaturen im Bereich von 130°C bis zum Siedepunkt des Reaktionsgemisches durchführt.

4. Verfahren nach Ansprüchen 1 bis 3, dadurch gekennzeichnet, daß man 0,1 bis 30 Mol-% Aluminiumtrichlorid, bezogen auf Mono-, Di- und/oder Polychloraromaten, einsetzt.

5. Verfahren nach Ansprüchen 1 bis 4, dadurch gekennzeichnet, daß man als Wasserspender ein Kristallwasser enthaltendes Salz einsetzt, das bei den angewendeten Temperaturen Wasser abgibt.

6. Verfahren nach Ansprüchen 1 bis 5, dadurch gekennzeichnet, daß man als Wasserspender eine organische Verbindung einsetzt, die Kristallwasser enthält und/oder eine organische Verbindung, die sich bei den angewendeten Temperaturen unter Wasserabspaltung zersetzt.

7. Verfahren nach Ansprüchen 1 bis 6, dadurch gekennzeichnet, daß man als Wasserspender Ameisensäure, Oxalsäure oder Oxalsäuredihydrat einsetzt.

8. Verfahren nach Ansprüchen 1 bis 7, dadurch gekennzeichnet, daß man Wasserspender in solchen Mengen einsetzt, daß während der Isomerisierung daraus 0,1 bis 30 Mol-% Wasser, bezogen auf Mono-, Di- und/oder Polychloraromaten, freigesetzt werden.

9. Verfahren nach Ansprüchen 1 bis 8, dadurch gekennzeichnet, daß Chlorwasserstoff mit einem Partialdruck von 0,01 bis 5 bar eingesetzt wird.

10. Verfahren nach Ansprüchen 1 bis 9, dadurch gekennzeichnet, daß man das nach der Isomerisierung vorliegende Reaktionsgemisch aufarbeitet, indem man zunächst filtriert oder dekantiert und dann aus der abgetrennten flüssigen Phase durch Destillation die einzelnen isomeren chlorierten Aromaten gewinnt.
